# EUROPEAN PATENT APPLICATION

(11) **EP 4 218 553 A1**
(43) Date of publication of application: **02.08.2023**
(21) Application number: 21879090.5
(22) Date of filing: 13.08.2021
(51) Int. Cl.: A61B 5/02

(54) **PPG SENSOR, ELECTRONIC DEVICE, AND WEARABLE DEVICE**

(30) Priority: 15.10.2020 CN 202022301597 U
(71) Applicant: GUANGDONG OPPO MOBILE TELECOMMUNICATIONS CORP., LTD., Dongguan, Guangdong 523860 (CN)
(72) Inventor: YAN, Rui, Dongguan, Guangdong 523860 (CN)
(74) Representative: Ipside
(86) International application number: PCT/CN2021/112370
(87) International publication number: WO 2022/078043

(57) **Abstract**

Provided are a PPG sensor, an electronic device, and a wearable device. The PPG sensor comprises a first light-emitting assembly (121) used for emitting a first optical signal; a second light-emitting assembly (124) used for emitting a second optical signal; and multiple photoelectric sensors (122) used for receiving the first optical signal and the second optical signal; wherein a distance between the first light-emitting assembly (121) and at least one photoelectric sensor (122) is greater than the minimum distance between the second light-emitting assembly (124) and the photoelectric sensors (122).

## Description

### CROSS REFERENCE TO RELATED APPLICATIONS

The present disclosure claims priority to Chinese Patent Application No. 202022301597.7, entitled "PPG SENSOR, ELECTRONIC DEVICE, AND WEARABLE DEVICE", filed on October 15, 2020, the entire contents of which are incorporated herein by reference.

### TECHNICAL FIELD

The present disclosure relates to wearable devices, particularly to a PPG sensor, an electronic device and a wearable device.

### BACKGROUND

The statements herein provide only background information relevant to the present disclosure and do not necessarily constitute exemplary prior art.

A wearable device may be provided with a PPG (Photo Plethysmo Graphy) sensor, and measure physiological parameter information such as heart rates, blood oxygen saturation and the like based on the PPG technology. There are two kinds of PPG detection technologies in view of layout of the PPG sensor, one is the transmission-mode detection technology, and the other is the reflection-mode detection technology. The PPG sensor applied in the wearable device generally exploits the reflection-mode detection technology to measure the physiological parameter information at the hand wrist. Current PPG sensors have the problem of inaccurate measuring results.

### SUMMARY

According to various embodiments of the present disclosure, a PPG sensor, an electronic device and a wearable device are provided.

According to some embodiments of the present disclosure, a PPG sensor is provided. The PPG sensor includes a first light-emitting assembly, a second light-emitting assembly and a plurality of photoelectric sensors. The first light-emitting assembly is configured to emit a first optical signal. The second light-emitting assembly is configured to emit a second optical signal. The plurality of photoelectric sensors are configured to receive the first optical signal and the second optical signal. A distance between the first light-emitting assembly and at least one of the plurality of photoelectric sensors is greater than a minimum one of the distances between the second light-emitting assembly and each photoelectric sensor.

With regard to the above-mentioned PPG sensor, by arranging the relative position relationships between the first light-emitting assembly, the second light-emitting assembly and the plurality of photoelectric sensors, and by arranging the distance between the first light-emitting assembly and at least one of the plurality of photoelectric sensors to be greater than the minimum one of the distances between the second light-emitting assembly and each photoelectric sensor, the distances between the first light-emitting assembly and the plurality of photoelectric sensors are different from the distances between the second light-emitting assembly and the plurality of photoelectric sensors, the distance requirements in application scenarios such as heart rate testing, blood pressure testing or blood oxygen testing etc. may be met. At the same time, the physiological parameter testing channels constituted by the first light-emitting assembly and each corresponding photoelectric sensor and the physiological parameter testing channels constituted by the second light-emitting assembly and each corresponding photoelectric sensor are different from each other, a suitable physiological parameter testing channel may be chosen based on the testing requirement of the physiological parameter, and the measurement precisions and the accuracies of the physiological parameters are enhanced.

According to some embodiments of the present disclosure, an electronic device is provided. The electronic device includes a housing and the PPG sensor as mentioned above. The housing defines a detection window. The PPG sensor is exposed from the detection window.

According to some embodiments of the present disclosure, a wearable device is provided. The wearable device includes a strap assembly and the electronic device as mentioned above. The strap assembly is configured to fix the electronic device to a human body.

Each of the above-mentioned wearable device and the electronic device may be provided with the PPG sensor, which may be configured to measure the physiological parameter information of the human body, the measurement accuracy of the PPG sensor is enhanced.

Details of one or more embodiments of the present disclosure will be illustrated in the following description and accompanying drawings. Other features, purposes and advantages of the present disclosure will be apparent from the specification, drawings and claims.

### BRIEF DESCRIPTION OF THE DRAWINGS

In order to more clearly illustrate technical solutions of the present disclosure or related art, the drawings required in the description of the embodiments or the related art will be briefly introduced below. The drawings in the following description are obviously only some embodiments of the present disclosure. For those of ordinary skills in the art, other drawings could be obtained based on these drawings without creative efforts.
Fig. 1 is a schematic structural perspective view of a wearable device according to some embodiments of the present disclosure.
Fig. 2 is a planar structural view of a wearable device according to some embodiments of the present disclosure.
Fig. 3a is a schematic view illustrating the transmission-mode PPG detection technology according to some embodiments of the present disclosure.
Fig. 3b is a schematic view illustrating the reflection-mode PPG detection technology according to some embodiments of the present disclosure.
Fig. 4 is a schematic view illustrating a first kind of frame structure of a PPG sensor according to some embodiments of the present disclosure.
Fig. 5 is a schematic view illustrating a second kind of frame structure of the PPG sensor according to some embodiments of the present disclosure.
Fig. 6 is a schematic view illustrating a third kind of frame structure of the PPG sensor according to some embodiments of the present disclosure.
Fig. 7 is a schematic view illustrating a fourth kind of frame structure of the PPG sensor according to some embodiments of the present disclosure.
Fig. 8 is a schematic view illustrating a fifth kind of frame structure of the PPG sensor according to some embodiments of the present disclosure.
Fig. 9 is a schematic view illustrating a sixth kind of frame structure of the PPG sensor according to some embodiments of the present disclosure.

### DETAILED DESCRIPTION

In order to make the purposes, technical solutions and advantages of the present disclosure clearer, the present disclosure will be described in further detail below with reference to the accompanying drawings and embodiments. It should be appreciated that, the specific embodiments described herein are intended only for explaining, not for limiting the present disclosure.

It should be appreciated that, the terms "first", "second", etc. used in the present disclosure may be used herein to describe various components, but these components are not limited to these terms. These terms are used only to distinguish a first component from another one. For instance, without departing from the scope of the present disclosure, the first light-emitting assembly may be referred to as the second light-emitting assembly. The second light-emitting assembly may be similarly referred to as the first light-emitting assembly. Both the first light-emitting assembly and the second light-emitting assembly are light-emitting assemblies, but are not a same light-emitting assembly.

In addition, the terms "first" and "second" are only for the purpose of description, and can't be construed as indicating or implying relative importance or implicitly indicating the number of technical features referred to. Therefore, the features preceded by "first" or "second" may explicitly or implicitly include at least one of the features. In the description of the present disclosure, "a plurality of" means at least two, such as two, three, etc., unless otherwise specifically defined. In the description of the present disclosure, "several" means at least one, such as one, two, etc., unless otherwise specifically defined.

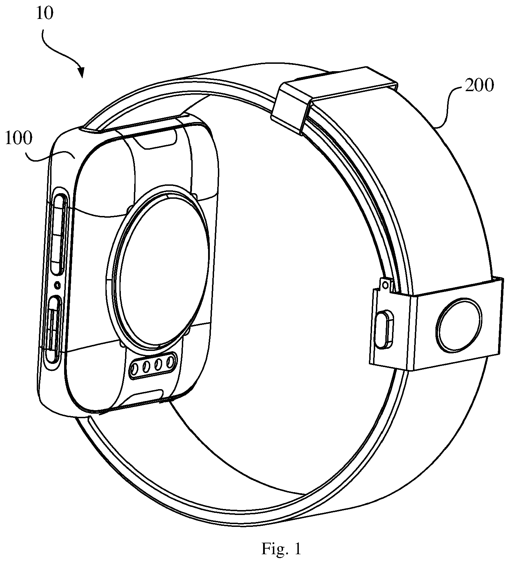

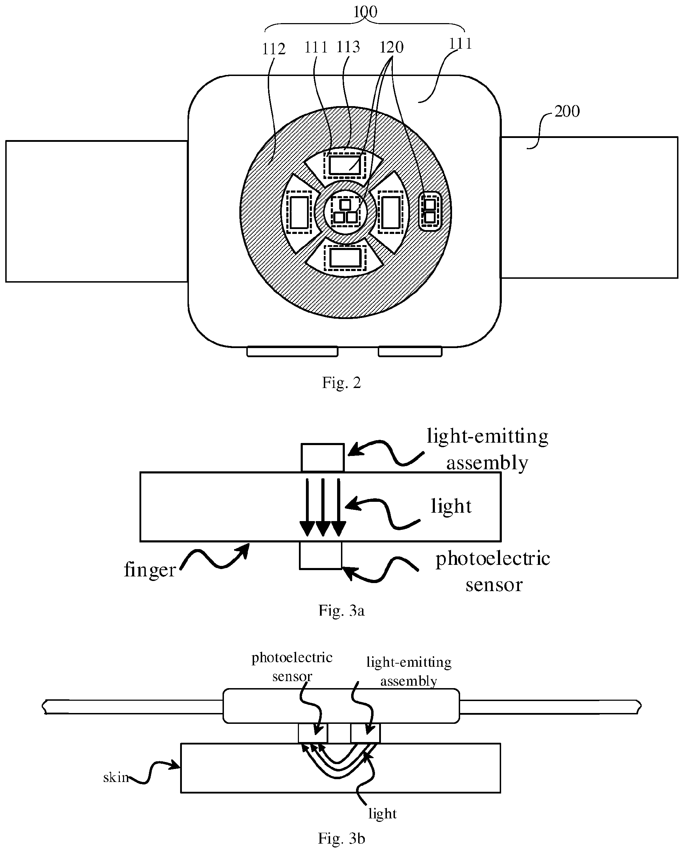

As shown in Fig. 1, in some embodiments, a wearable device 10 includes an electronic device 100 and a strap assembly 200. The electronic device 100 is mounted to the strap assembly 200. The electronic device 100 can be worn to a user's wrist via the strap assembly 200. As shown in Fig. 2, the electronic device 100 includes a housing 110 and electronic components such as circuit boards and batteries that are arranged inside the housing 110. The housing 110 defines a mounting cavity. The electronic components such as circuit boards or batteries etc. are arranged in the mounting cavity. The housing 110 may be made of non-metallic materials such as plastic, rubber, silica gel, wood, ceramic or glass. The housing 110 may also be made of metal materials such as stainless steel, aluminum alloy or magnesium alloy. The housing 110 may also be a metal injection molded part. Metal materials are used to ensure the structural rigidity of the housing 110. Bumps, grooves, threaded holes and other structures that are configured for assembly and positioning are formed by injection molding on the inner surface of the metal piece.

A detection window 111 is defined in the housing 110. The detection window 111 is configured for light transmission, to transmit optical signals emitted and reflected by the electronic components built in the housing 110, thereby enabling detection of vital signs such as human heart rate and blood oxygen saturation etc. The electronic components are for example PPG sensors 120. There are two kinds of PPG detection technologies in view of layout of the PPG sensor 120: one is the transmission-mode detection technology, the other is the reflection-mode detection technology. As shown in Fig. 3a, the transmission-mode detection technology may trace and record, based on differences of intensities of the transmitted light, changes of a vascular volume during a cardiac cycle, and obtain the heart rate, the blood oxygen saturation or etc. As shown in Fig. 3b, the reflection-mode detection technology may trace and record, based on the differences of intensities of the reflected light, changes of the vascular volume during a cardiac cycle, and obtain vital signs such as heart rates, blood oxygen saturation, etc. By incorporating the PPG sensor 120 provided in the present disclosure to the wearable device 10, the user may conveniently detect his/her heart rates and blood oxygen saturation, etc. anytime and anywhere. In this way, not only functions of the wearable electronic device are enriched, but also health needs of the user are greatly satisfied.

In some embodiments, an opaque baffle wall 112 and a transparent window 113 are also provided in the housing 110. The size matching between the opaque baffle wall 112 and the detection window 111 prevents optical crosstalk between the light-emitting assemblies of the PPG sensor 120 and the photoelectric sensor. The transparent window 113 is overlayed on and covers the detection window 111 for waterproof and dustproof, and increases light transmission of the PPG sensor 120.

In some embodiments, the wearable device 10 is a smartwatch or a bracelet. The electronic components such as the battery, the circuit board, the display screen module and the PPG sensor 120 etc. are arranged in the mounting cavity. Electronic components such as a processor, a storage unit and a communication module etc. of the wearable device 10 may be integrated on the circuit board. The battery may supply power to the circuit board, the display screen module and other electronic components.

The housing 110 may have a shape of a substantially rectangular box. Four corners of the rectangle may be chamfered into circular arc transitions, such that the wearable device 10 has a better appearance characteristic. In some embodiments, the housing 110 may have a shape of a circular frame. The side face of the housing 110 may be provided with a mating structure for mounting the strap assembly 200. The strap assembly 200 is able to be reliably connected to the housing 110 through the mating structure of the housing 110, such that the electronic device 100 is reliably worn to a hand of the user. In some embodiments, the strap assembly 200 may also be relatively easily detached from the housing 110, thus the user is allowed to easily replace the strap assembly 200. For example, the user may purchase multiple styles of strap assemblies 200, and change the strap assembly 200 based on the using scenario, thereby enhancing the convenience of use. For example, the user may use a more formal strap assembly 200 for formal occasions and a casual style strap assembly 200 for casual entertainment occasions.

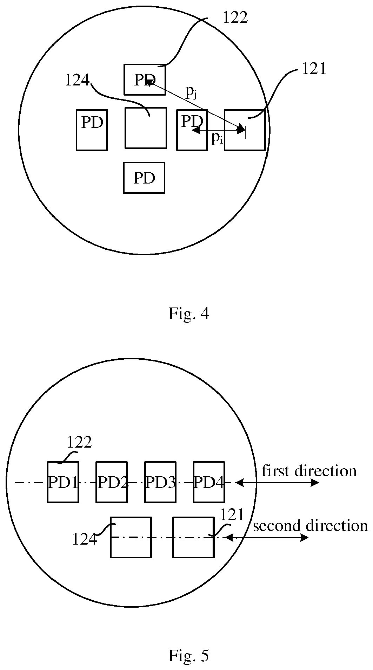

In some embodiments, as shown in Fig. 4, the PPG sensor includes a first light-emitting assembly 121, a plurality of photoelectric sensors 122 and a second light-emitting assembly 124. The first light-emitting assembly 121 may be configured to receive a drive signal to emit a first optical signal. The second light-emitting assembly 124 may be configured to receive a drive signal to emit a second optical signal. The first optical signal and the second optical signal may be the same or different. The PPG sensor may control the types of optical signals emitted by the first light-emitting assembly 121 and the second light-emitting assembly 124 according to the measurement requirement. Each of the first optical signal and the second optical signal may include at least one selected from the group consisting of the red light, the infrared light and the green light. It should be noted that, the measurement requirement includes, but is not limited to, heart rate measurement, blood pressure measurement and blood oxygen saturation measurement.

Each photoelectric sensor 122 may operate at a zero-bias state. That is, each photoelectric sensor 122 may be a photodiode PD operating in a photovoltaic mode. Each photoelectric sensor 122 is configured to receive the first optical signal emitted by the first light-emitting assembly 121 and the second optical signal emitted by the second light-emitting assembly 124, and may convert the received first optical signal and second optical signal into electrical signals.

The distance between the first light-emitting assembly 121 and the at least one photoelectric sensor 122 is greater than the minimum one of the distances between the second light-emitting assembly 124 and each of the photoelectric sensors 122. Specifically, the distance between the second light-emitting assembly 124 and any one of the photoelectric sensors 122 may be noted as qᵢ. The minimum distance of the distances between the second light-emitting assembly 124 and each photoelectric sensor 122 may be noted as qₘᵢₙ. The distance between the first light-emitting assembly 121 and the at least one photoelectric sensor 122 is also noted as p₁-pₙ. In other words, any one of the distances p₁-pₙ is greater than the minimum distance qₘᵢₙ. The distances between the first light-emitting assembly 121 and the plurality of photoelectric sensors 122 and the distances between the second light-emitting assembly 124 and the plurality of photoelectric sensors 122 may meet the distance requirements in application scenarios such as heart rate testing, blood pressure testing, blood oxygen testing etc., and the measurement accuracy of the heart rate measurement and the blood oxygen saturation measurement are enhanced.

The layout of the first light-emitting assembly 121, the second light-emitting assembly 124 and the plurality of photoelectric sensors 122 in the PPG sensor provided according to some embodiments of the present disclosure may meet the distance requirements between each photoelectric sensor 122 and the first light-emitting assembly 121 and the distance requirements between each photoelectric sensor 122 and the second light-emitting assembly 124 in different application scenarios. For example, when the first light-emitting assembly 121 is a two-in-one LED assembly emitting both the red light and the infrared light, and the second light-emitting assembly 124 is a green-light LED, the measurement of the heart rate information may be achieved by measuring the emitted light intensity between the second light-emitting assembly 124 and the photoelectric sensor 122 that have a shortest distance between them, the measurement of the blood oxygen saturation information may be further achieved by measuring the emitted light intensity between the first light-emitting assembly 121 and the photoelectric sensor 122 that have a longest distance between them. Therefore, the layout of the first light-emitting assembly 121, the second light-emitting assembly 124 and the plurality of photoelectric sensors 122 of the PPG sensor provided according to some embodiments of the present disclosure may take into accounts of and balance the measurement requirements of the heart rate measurement, the blood pressure testing and the blood oxygen saturation measurement, which can in turn improve the measurement accuracies of the heart rate measurement and the blood oxygen saturation measurement.

Further, since the number of the photoelectric sensors 122 are multiple, the plurality of photoelectric sensors 122 may constitute a corresponding physiological parameter measurement channel with each of the first light-emitting assembly 121 and the second light-emitting assembly 124. When the PPG sensor 120 is worn, each photoelectric sensor 122 corresponds to a different biological skin tissue region. The depths of the biological skin tissues penetrated by the first optical signal and the second optical signal are different, the reflected light intensities of the first optical signal and the second optical signal acquired by each photoelectric sensor 122 are also different. Therefore, a plurality of physiological parameter measurement channels of the PPG sensor may correspondingly measure a plurality of physiological parameters at different biological skin tissue positions and different depths of the user, the testing precisions and accuracies of the physiological parameters are enhanced.

In some embodiments, the first optical signal may include the red light and/or the infrared light. The second optical signal includes the green light. Specifically, the first light-emitting assembly 121 may include any of an LED assembly with a red-light LED or an infrared-light LED, a two-in-one LED assembly emitting both the red light and the infrared light, a two-in-one LED assembly emitting both the green light and the infrared light LED, a two-in-one LED assembly emitting both the green light and the infrared light and a three-in-one LED assembly emitting the green light, the red light and the infrared light. The second light-emitting assembly 124 may include any of a green-light LED, a two-in-one LED assembly emitting both the green light and the infrared light, a two-in-one LED assembly emitting both the green light and the infrared light and a three-in-one LED assembly emitting the green light, the red light and the infrared light.

Each photoelectric sensor 122 constitutes a first blood oxygen measurement channel with the first light-emitting assembly 121. Each photoelectric sensor 122 constitutes a heart rate measurement channel with the second light-emitting assembly 124. In other words, when the number of the photoelectric sensor 122 is n, n is greater than or equal to 2, the plurality of photoelectric sensors 122 may be noted as PD1, PD2, PD3, ..., and PDn respectively. n first blood oxygen measurement channels may be constituted by the first light-emitting assembly 121 with each photoelectric sensor 122 correspondingly. It should be appreciated that, each first blood oxygen measurement channel corresponds to a photoelectric sensor 122. Accordingly, n heart rate measurement channels are constituted by the second light-emitting assembly 124 with each photoelectric sensor 122 correspondingly. It should be appreciated that, each heart rate measurement channel corresponds to a photoelectric sensor 122.

When the first light-emitting assembly 121 is a two-in-one LED assembly emitting both the red light and the infrared light, and the second light-emitting assembly 124 is a green-light LED, the intensity of reflected light is acquired by the PPG sensor based on the heart rate measurement channel with the shortest distance, and thereby enabling testing of the heart rate information. The heart rate measurement channel with the minimum distance may be constituted by the second light-emitting assembly 124 and the photoelectric sensor 122 spaced apart from each other at a minimum distance. Specifically, the minimum distance qₘᵢₙ of the distances between the second light-emitting assembly 124 and each photoelectric sensor 122 ranges from 4mm to 5mm. The intensity of the reflected light may be acquired by the PPG sensor based on a long-distance blood oxygen measurement channel, enabling the testing of the blood oxygen saturation information. The long-distance blood oxygen measurement channel may be constituted by the first light-emitting assembly 121 and a photoelectric sensor 122 between which the distance is greater than the minimum distance qₘᵢₙ. Specifically, the distance between the first light-emitting assembly 121 and the at least one photoelectric sensor 122 is ranged from 7mm to 9mm, to meet the requirement of being greater than the minimum distance qₘᵢₙ.

When the PPG sensor 120 is worn, each photoelectric sensor 122 corresponds to a different biological skin tissue region. When different first blood oxygen measurement channels acquire the first optical signals, the depth of the biological skin tissues penetrated by the first optical signal is different, the light intensity of the first optical signal acquired by each photoelectric sensor 122 may also be different. Therefore, the plurality of first blood oxygen measurement channels of the PPG sensor 120 may correspondingly measure a plurality of first blood oxygen testing results of different biological skin tissue positions and different depths of the user. In this way, the effect of venous pulse etc. on the blood oxygen measurement may be reduced, the measurement error of the blood oxygen saturation information may be reduced, and the testing precision and the accuracy of the blood oxygen saturation may be increased. At the same time, when different heart rate measurement channels acquire the second optical signals, the depths of the biological skin tissues penetrated by the second optical signals are different, the light intensities of the second optical signals acquired by each photoelectric sensor 122 are also different. Therefore, the plurality of heart rate measurement channels of the PPG sensor 120 may correspondingly measure the plurality of heart rate testing results of different biological skin tissue positions and different depths of the user, further enhancing the testing precision and the accuracy of the heart rate information.

The layout of the first light-emitting assembly 121, the second light-emitting assembly 124 and the plurality of photoelectric sensors 122 in the PPG sensor 120 provided according to some embodiments of the present disclosure may take into accounts and balance the measurement requirements of the heart rate measurement and the blood oxygen saturation measurement. At the same time, the number of the heart rate measurement channels in the PPG sensor is multiple, the number of the first blood oxygen measurement channels in the PPG sensor is also multiple, and the intensity of the reflected light received by each measurement channel is also different. In this way, the measurement precision and accuracy of the heart rate measurement and the blood oxygen saturation measurement may be enhanced.

In some embodiments, when the first light-emitting assembly 121 is a two-in-one LED assembly emitting the red light and the infrared light, or the first light-emitting assembly 121 is an LED assembly emitting the red light or the infrared light, and/or when the second light-emitting assembly 122 is a two-in-one LED assembly emitting the red light and the infrared light, or when the second light-emitting assembly 122 is an LED assembly emitting the red light or the infrared light, detection of the blood pressure information may also be enabled by the PPG sensor based on the intensity of the reflected light acquired by each photoelectric sensor 122, thereby expanding the functionality of the PPG sensor.

The specific way of arrangement of the PPG sensor 120 provided according to some embodiments of the present disclosure may be detailed in conjunction with the accompanying drawings below. It should be noted that, in some embodiments of the present disclosure, as an example, the profile of the PPG sensor 120 is illustrated to have a circular shape. In fact, the profile of the PPG sensor 120 may also have other shapes, such as oval or elongated strip etc.

As shown in Fig. 4, in some embodiments, the distance between one of the at least two photoelectric sensors 122 and the first light-emitting assembly 121 is different from the distance between another one of the at least two photoelectric sensors 122 and the first light-emitting assembly 121. In other words, the distances between the first light-emitting assembly 121 and the at least two photoelectric sensors 122 are different. If any two photoelectric sensors 122 are denoted as PDi and PDj respectively, the distance between the first light-emitting assembly 121 and the photoelectric sensor PDi may be denoted as pᵢ, the distance between the first light-emitting assembly 121 and the photoelectric sensor PDj may be denoted as pⱼ. 1 ≤ i ≠ j ≤ n, and pi ≠ pⱼ. For example, if the number of the plurality of photoelectric sensors 122 is 4, the distances between the first light-emitting assembly 121 and the four photoelectric sensors 122 may be denoted as p₁, p₂, p₃ and p₄ respectively. Then the distances between the first light-emitting assembly 121 and the at least two photoelectric sensors 122 being different may be construed as, any three distances of pi, p₂, p₃ and p₄ are the same, or any two distances of p₁, p₂, p₃ and p₄ are the same, or any three distances of p₁, p₂, p₃ and p₄ are different from each other, or the four distances of p₁, p₂, p₃ and p₄ are different from each other. The distance from the first light-emitting assembly 121 to any of the plurality of photoelectric sensors 122 may be construed as the distance from the center position of the first light-emitting assembly 121 to the center position of the same photoelectric sensor 122. The center position may be construed as the geometric center of each assembly and device.

Since the distances between the first light-emitting assembly 121 and the at least two photoelectric sensors 122 are different, for a same optical signal, the light intensities or luminous fluxes received by the photoelectric sensors 122 corresponding to different distances may also be different. In other words, the intensities of light received through the corresponding first blood oxygen measurement channels of the PPG sensor 120 corresponding to the photoelectric sensors 122 may also be different. In addition, when the PPG sensor 120 is worn, each photoelectric sensor 122 corresponds to different biological skin tissue regions. When the first optical signals are acquired by different first blood oxygen measurement channels, the depths of the biological skin tissues penetrated by the first optical signals are different, the light intensity of the first optical signal acquired by each photoelectric sensor 122 is also different. Therefore, the plurality of first blood oxygen measurement channels of the PPG sensor 120 may correspondingly measure a plurality of first blood oxygen testing results corresponding to different biological skin tissue positions and different depths of the user. In this way, the influence of venous pulse etc. on the blood oxygen measurement may be reduced, the measurement error of the blood oxygen saturation information may be reduced, and the testing precision and the accuracy of the blood oxygen saturation may be increased.

In the PPG sensor 120 provided according to the above-mentioned embodiments, by setting the layout way of the first light-emitting assembly 121 and the plurality of photoelectric sensors 122, a plurality of different first blood oxygen measurement channels may be formed. In this way, the testing requirements of blood oxygen saturation in different scenarios are satisfied, the testing accuracy of the blood oxygen saturation may at the same time be enhanced. In this way, the physiological parameters of the wearer are measured by using the plurality of first blood oxygen measurement channels (the multiplex light intensities), the error of the testing results may be reduced, and the accuracy of the detection result may be enhanced.

In some embodiments, when the first light-emitting assembly 121 is a three-in-one LED assembly emitting the red light, the infrared light and the green light, a plurality of first blood oxygen measurement channels and a plurality of heart rate measurement channels are formed. The processing module 123 may obtain, according to the first blood oxygen testing results measured by the plurality of first blood oxygen measurement channels, the blood oxygen saturation information. The processing module 123 may obtain, according to the heart rate testing results measured by the plurality of heart rate measurement channels, the heart rate information. Therefore, the accuracies of the detection results are enhanced.

As shown in Fig. 5, in some embodiments, the plurality of photoelectric sensors 122 are arranged along a same straight line at intervals. The first light-emitting assembly 121 and the second light-emitting assembly 124 are located on a same side of the straight line. For example, the plurality of photoelectric sensors 122 include PD1, PD2, PD3 and PD4. PD1, PD2, PD3 and PD4 are arranged along a straight line extending along a first direction. The first light-emitting assembly 121 and the second light-emitting assembly 124 are located on a same side of the straight line. For example, the first light-emitting assembly 121 and the second light-emitting assembly 124 may be arranged along a straight line extending in a second direction. The first direction and the second direction are perpendicular to each other. In some embodiments, the first direction and the second direction are the same direction, and a straight line extending in the first direction and a straight line extending along the second direction do not coincide. In some embodiments, the second direction may make an acute angle with the first direction.

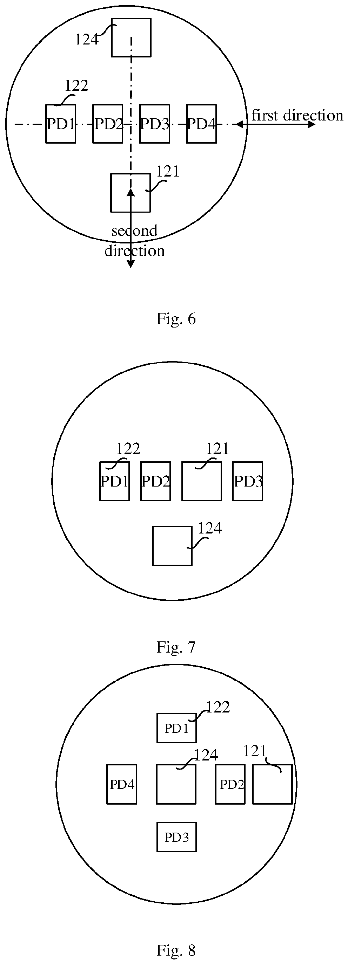

As shown in Fig. 6, in some embodiments, the plurality of photoelectric sensors 122 may be arranged along a same straight line at intervals. The first light-emitting assembly 121 and the second light-emitting assembly 124 are located on either side of the straight line respectively. For example, the plurality of photoelectric sensors 122 include PD1, PD2, PD3 and PD4. PD1, PD2, PD3 and PD4 are arranged along a straight line extending along a first direction at intervals. The first light-emitting assembly 121 may be located at a first side of the straight line, the second light-emitting assembly 124 may be located at a second side of the straight line. A distance from the second light-emitting assembly 124 to the straight line is less than a distance from the first light-emitting assembly 121 to the straight line. The first light-emitting assembly 121 and the second light-emitting assembly 124 may be arranged along a straight line, which may be perpendicular to the straight line where PD1, PD2, PD3 and PD4 are located, or may make an inclined angle with the straight line where PD1, PD2, PD3 and PD4 are located.

It should be noted that, the plurality of photoelectric sensors 122 may be arranged in the same straight line at a regular interval or at irregular intervals. The lines connecting centers of any two of the plurality of photoelectric sensors 122 may be in the same straight line, but a certain engineering error may be allowed.

As shown in Fig. 7, in some embodiments, the first light-emitting assembly 121 and the plurality of photoelectric sensors 122 are arranged in a same straight line at intervals. The second light-emitting assembly 124 is not on the straight line. For example, the plurality of photoelectric sensors 122 may include PD1, PD2 and PD3. The first light-emitting assembly 121 may be arranged between PD1 and PD2, or between PD2 and PD3. The second light-emitting assembly 124 may not be on the straight line. It should be noted that, the first light-emitting assembly 121 and the plurality of photoelectric sensors 122 may be arranged on the same straight line at a regular interval or at irregular intervals. The lines connecting centers of any two of the plurality of photoelectric sensors 122 may be located on the same straight line, but a certain engineering error may be allowed.

In some embodiments, the plurality of photoelectric sensors 122 may be arranged in an array. The array may be a rectangular array or an annular array. The second light-emitting assembly 124 is located at the central position of the array. The array is located in an area S. The first light-emitting assembly 121 is arranged outside of the area S. For example, as shown in Fig. 8, the plurality of photoelectric sensors 122 include four photoelectric sensors 122. The four photoelectric sensors 122 may be noted as PD1, PD2, PD3 and PD4 respectively. PD1, PD2, PD3 and PD4 may be arranged in an annular array. The array is located in the area S. The first light-emitting assembly 121 is arranged outside of the area S. The second light-emitting assembly 124 is located at the central position of the array. In other words, the distance from the second light-emitting assembly 124 to any of PD1, PD2, PD3 and PD4 is the same. The distance from the first light-emitting assembly 121 to PD2 is equal to the distance from the first light-emitting assembly 121 to PD3. Specifically, the distance from the second light-emitting assembly 124 to any of PD1, PD2, PD3 and PD4 ranges from 4mm to 5mm, such that the heart rate information is tested by the heart rate measurement channel constituted by the second light-emitting assembly 124 and each PD, the accuracy of the heart rate information testing is further enhanced, and the power consumption of the PPG sensor 120 is reduced. The distance from the first light-emitting assembly 121 to any of PD2 and PD3 ranges from 7mm to 9mm, such that the blood oxygen saturation information is tested by the blood oxygen measurement channel constituted by the first light-emitting assembly 121 and each PD, the accuracy of the blood oxygen saturation information testing is further enhanced.

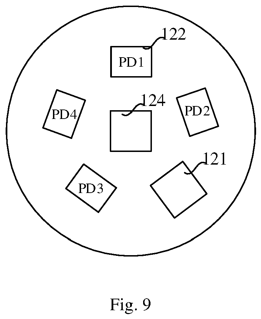

As shown in Fig. 9, in some embodiments, the first light-emitting assembly 121 and the plurality of photoelectric sensors 122 are arranged in an array. The second light-emitting assembly 124 is arranged at the central position of the array. In other words, the first light-emitting assembly 121 and each photoelectric sensor 122 are used as a part of the array. The relative positions of the first light-emitting assembly 121 with respect to each photoelectric sensor 122 are not further limited. The plurality of photoelectric sensors 122 may include four photoelectric sensors 122. The four photoelectric sensors 122 may be noted as PD1, PD2, PD3 and PD4 respectively. PD1, PD2, PD3, PD4 and the first light-emitting assembly 121 are arranged in an annular array. The second light-emitting assembly 124 is located at the central position of the annular array, such that the distances from the second light-emitting assembly 124 to any of PD1, PD2, PD3 and PD4 are the same.

In some embodiments, the plurality of photoelectric sensors 122 may include four photoelectric sensors 122. The four photoelectric sensors 122 may be noted as PD1, PD2, PD3 and PD4 respectively. PD1, PD2, PD3, PD4 and the first light-emitting assembly 121 are arranged in a rectangular array. The second light-emitting assembly 124 is located at the central position of the rectangle array, such that the distances from the second light-emitting assembly 124 to any of PD1, PD2, PD3 and PD4 are the same.

In the afore-mentioned embodiments of the PPG sensor 120, by setting the layout manner of the first light-emitting assembly 121, the second light-emitting assembly 124 and the plurality of photoelectric sensors 122, a plurality of different first blood oxygen measurement channels and heart rate measurement channels may be formed. Therefore, testing requirements of blood oxygen saturation and heart rates in different scenarios are satisfied, and the testing accuracies of the blood oxygen saturation and the heart rate may at the same time be increased. In this way, the physiological parameters of the wearer may be tested by using the plurality of first blood oxygen measurement channels (multiplex light intensities) and the plurality of heart rate measurement channels (multiplex light intensities), the errors of the testing results may be reduced, and the accuracies of the testing results may be increased.

In some embodiments, the second light-emitting assembly 124 may emit a first optical signal and a second optical signal. The first optical signal is configured to measure the blood oxygen saturation information. The second optical signal is configured to measure the heart rate information. The first optical signal includes at least one kind of light selected from the group consisting of the red light and the infrared light. As an example, the second light-emitting assembly 124 may include any of a two-in-one LED assembly emitting the green light and the infrared light, a two-in-one LED assembly emitting the green light and the infrared light, and a three-in-one LED assembly emitting the green light, the red light and the infrared light. Each photoelectric sensor 122 constitutes a heart rate measurement channel with the second light-emitting assembly 124. Each photoelectric sensor 122 constitutes a second blood oxygen measurement channel with the second light-emitting assembly 124. In other words, when the number of the plurality of photoelectric sensors 122 is n, the number of the corresponding first blood oxygen measurement channels is n, the number of the corresponding second blood oxygen measurement channels is n, the number of the corresponding heart rate measurement channels is n. In other words, the number of the measurement channels configured to test the blood oxygen saturation is 2n.

As an example for illustration, the first light-emitting assembly 121 is a two-in-one LED assembly emitting the red light and the infrared light, the second light-emitting assembly 124 is a green-light LED, the plurality of photoelectric sensors 122 include PD1, PD2, PD3 and PD4. The first light-emitting assembly 121, the second light-emitting assembly 124, PD1, PD2, PD3 and PD4 may constitute a plurality of first blood oxygen measurement channels and a plurality of heart rate measurement channels. Specifically, a first blood oxygen measurement channel 1 is a combination of the first light-emitting assembly 121 and PD1; a first blood oxygen measurement channel 2 is a combination of the first light-emitting assembly 121 and PD2; a first blood oxygen measurement channel 3 is a combination of the first light-emitting assembly 121 and PD3; a first blood oxygen measurement channel 4 is a combination of the first light-emitting assembly 121 and PD4; a heart rate measurement channel 1 is a combination of the second light-emitting assembly 124 and PD1; a heart rate measurement channel 2 is a combination of the second light-emitting assembly 124 and PD2; a heart rate measurement channel 3 is a combination of the second light-emitting assembly 124 and PD3; and a heart rate measurement channel 4 is a combination of the second light-emitting assembly 124 and PD4.

Based on the above-mentioned example, the PPG sensor 120 may constitute four first blood oxygen measurement channels and four heart rate measurement channels. Specifically, the PPG sensor 120 may further include a processing module. The processing module is connected to each of the first light-emitting assembly 121, the second light-emitting assembly 124 and each photoelectric sensor 122 respectively. The blood oxygen testing result may be obtained, by the processing module, based on the testing result of each first blood oxygen measurement channel. The heart rate testing result may be obtained, by the processing module, based on the testing results of the heart rate measurement channels.

In some embodiments, the processing module may specifically include an analog front-end processing unit and a processor. The analog front-end unit may be respectively connected to each of the first light-emitting assembly 121 and the second light-emitting assembly 124, and to each photoelectric sensor 122. The analog front-end unit may drive the first light-emitting assembly 121 and the second light-emitting assembly 124 to emit the first optical signal, and receive the corresponding photoelectric signal acquired by each of the photoelectric sensor 122. The analog front-end unit may process the received plurality of photoelectric signals, and correspondingly obtain the testing result corresponding to each of the blood oxygen measurement channels and each of the heart rate measurement channels. The processor is connected to the analog front-end processing unit. The processor may obtain blood oxygen testing results according to the testing result corresponding to each blood oxygen measurement channel. The processor may also obtain heart rate testing results according to the testing results corresponding to the heart rate measurement channels. Specifically, the processor may obtain the testing result with the maximum value based on the four testing results of the four first blood oxygen measurement channels, and obtain the blood oxygen testing result based the testing result with the maximum value. In this way, it is possible to remove the blood oxygen testing results that are caused to be relatively lower due to venous pulsation, etc. The possibility of testing results affected by the venous pulsation may be further reduced, and the measure precision may be enhanced. The heart rate testing result may be obtained, by the processor, according to the average value of the four testing results of the four heart rate measurement channels. The errors of the detection results may be reduced, and the accuracy of the detection results may be enhanced.

It should be noted that, in some embodiments of the present disclosure, the blood oxygen testing results may be construed as the blood oxygen saturation information, and the heart rate testing results may be construed as the heart rate information.

As an example for illustration, the first light-emitting assembly 121 is a two-in-one LED assembly emitting the red light and the infrared light, the second light-emitting assembly 124 is a three-in-one LED assembly including the red-light LED, the infrared-light LED and the green-light LED, the plurality of photoelectric sensors 122 include PD1, PD2, PD3 and PD4. The first light-emitting assembly 121, the second light-emitting assembly 124, PD1, PD2, PD3 and PD4 may constitute a plurality of first blood oxygen measurement channels and a plurality of heart rate measurement channels. Specifically, a first blood oxygen measurement channel 1 is a combination of the first light-emitting assembly 121 and PD1; a first blood oxygen measurement channel 2 is a combination of the first light-emitting assembly 121 and PD2; a first blood oxygen measurement channel 3 is a combination of the first light-emitting assembly 121 and PD3; a first blood oxygen measurement channel 4 is a combination of the first light-emitting assembly 121 and PD4; a second blood oxygen measurement channel 1 is a combination of the red light and the infrared light of the second light-emitting assembly 124 and PD1; a second blood oxygen measurement channel 2 is a combination of the red light and the infrared light of the second light-emitting assembly 124 and PD2; a second blood oxygen measurement channel 3 is a combination of the red light and the infrared light of the second light-emitting assembly 124 and PD3; a second blood oxygen measurement channel 4 is a combination of the red light and the infrared light of the second light-emitting assembly 124 and PD4; a heart rate measurement channel 1 is a combination of the green light of the second light-emitting assembly 124 and PD1; a heart rate measurement channel 2 is a combination of the green light of the second light-emitting assembly 124 and PD2; a heart rate measurement channel 3 is a combination of the green light of the second light-emitting assembly 124 and PD3; and a heart rate measurement channel 4 is a combination of the green light of the second light-emitting assembly 124 and PD4.

Based on the above-mentioned example, the PPG sensor 120 may constitute four heart rate measurement channels, four first blood oxygen measurement channels and four second blood oxygen measurement channels. In other words, eight blood oxygen measurement channels may be constituted. Based on the four heart rate measurement channels and the eight blood oxygen measurement channels, the measuring accuracies and precision of the heart rate information and the blood oxygen saturation information may be enhanced. During the process of obtaining the blood oxygen testing results by the processing module, the processing module may first obtain the testing result with the maximum value from the eight blood oxygen measurement channels, and obtain the blood oxygen testing result according to the testing result with the maximum value.

In embodiments of the present disclosure, by arranging the relative position relationships of the first light-emitting assembly 121, the second light-emitting assembly 124 and the plurality of photoelectric sensors 122 of the PPG sensor, the light fluxes received by the photoelectric sensors 122 positioned at different distances from a same first light-emitting assembly 121 will accordingly be different, the light fluxes received by the photoelectric sensors 122 positioned at different distances from a same second light-emitting assembly 124 will accordingly be different. In other words, the light fluxes received via different first blood oxygen measurement channels of the PPG sensor 120 are accordingly different, the light fluxes received via different heart rate measurement channels of the PPG sensor 120 are accordingly different. In addition, when the PPG sensor 120 is worn, each photoelectric sensor 122 corresponds to different biological skin tissue regions. When different first blood oxygen measurement channels acquire the first optical signals, the depth of the biological skin tissues penetrated by the first optical signals are different. The light flux of the first optical signal acquired by each photoelectric sensor 122 is also different. When different heart rate measurement channels acquire the second optical signals, the depth of the biological skin tissues penetrated by the second optical signals are different. The light flux of the second optical signal acquired by each photoelectric sensor 122 is also different. Therefore, the plurality of heart rate measurement channels of the PPG sensor 120 may correspondingly measure a plurality of first blood oxygen measurement channels and heart rate testing results at different biological skin tissue positions and at different depths of the user.

According to some embodiments of the present disclosure, an electronic device is also provided. The electronic device may include the PPG sensor of any of the above-mentioned embodiments. The PPG sensor may measure the physiological parameter information of a human body, the measurement accuracy thereof is enhanced.

According to some embodiments of the present disclosure, a wearable device is further provided. The wearable device may include a strap assembly and the electronic device according to any of the above-mentioned embodiments. The wearable device may measure the physiological parameter information of the human body, and enhance the measurement accuracy thereof.

The above-mentioned embodiments only illustrate a few implementations of the present disclosure, the description thereof is relatively specific and detailed, but they could not be construed as restrictions on the scope of the patent application. It should be pointed out that, for those of ordinary skills in the art, without departing from the concept of the present disclosure, several modifications and improvements could be made, and these all fall within the protection scope of the present disclosure. Therefore, the protection scope of this patent application should be subject to the appended claims.

## Claims

1. A photo plethysmo graphy (PPG) sensor, comprising:
a first light-emitting assembly, configured to emit a first optical signal;
a second light-emitting assembly, configured to emit a second optical signal; and
a plurality of photoelectric sensors, configured to receive the first optical signal and the second optical signal;
wherein a distance between the first light-emitting assembly and at least one of the plurality of photoelectric sensors is greater than a minimum one of distances between the second light-emitting assembly and each of the plurality of photoelectric sensors.

2. The PPG sensor as claimed in claim 1, wherein
a distance between one of at least two of the plurality of photoelectric sensors and the first light-emitting assembly is different from a distance between another one of the at least two of the plurality of photoelectric sensors and the first light-emitting assembly.

3. The PPG sensor as claimed in claim 1, wherein
the plurality of photoelectric sensors are arranged along a same straight line at intervals; and
the first light-emitting assembly and the second light-emitting assembly are located at a same side of the straight line.

4. The PPG sensor as claimed in claim 1, wherein
the plurality of photoelectric sensors are arranged along a same straight line at intervals; and
the first light-emitting assembly is located at one side of the straight line, the second light-emitting assembly is located at the other side of the straight line.

5. The PPG sensor as claimed in claim 1, wherein
the first light-emitting assembly and the plurality of photoelectric sensors are arranged along a same straight line at intervals; and
the second light-emitting assembly is not on the straight line.

6. The PPG sensor as claimed in claim 1, wherein
the plurality of photoelectric sensors are arranged in an array;
the second light-emitting assembly is located at a central position of the array; and
the first light-emitting assembly is located outside of an area where the array is located in.

7. The PPG sensor as claimed in claim 1, wherein
the first light-emitting assembly and the plurality of photoelectric sensors are arranged in an array; and
the second light-emitting assembly is located at a central position of the array.

8. The PPG sensor as claimed in any of claims 6 or 7, wherein
the array comprises a rectangular array or an annular array.

9. The PPG sensor as claimed in claim 1, wherein
the second light-emitting assembly is further configured to emit the first optical signal, the first optical signal comprises at least one selected from the group consisting of red light and infrared light.

10. The PPG sensor as claimed in claim 1, wherein
each of the plurality of photoelectric sensors constitutes a first blood oxygen measurement channel with the first light-emitting assembly;
each of the plurality of photoelectric sensors constitutes a heart rate measurement channel with the second light-emitting assembly;
the PPG sensor further comprises a processing module,
the processing module is connected to the first light-emitting assembly, the second light-emitting assembly, and each of the plurality of photoelectric sensors,
the processing module is configured to obtain, based on testing results of each first blood oxygen measurement channel, a blood oxygen testing result, and
the processing module is configured to obtain, based on testing results of each heart rate testing channel, a heart rate testing result.

11. The PPG sensor as claimed in claim 10, wherein
the processing module is further configured to drive the second light-emitting assembly to emit the first optical signal;
each of the plurality of photoelectric sensors constitutes a second blood oxygen testing channel with the second light-emitting assembly;
the processing module is further configured to obtain, based on a maximum one of testing results of each first blood oxygen measurement channel and each second blood oxygen testing channel, the blood oxygen testing result.

12. The PPG sensor as claimed in claim 10, wherein
the processing module is further configured to obtain testing results measured by each heart rate measurement channel, and obtain the heart rate testing result based on an average value of a plurality of the testing results.

13. The PPG sensor as claimed in claim 10, wherein
the processing module comprises:
an analog front-end processing unit, connected to the first light-emitting assembly, the second light-emitting assembly and each of the plurality of photoelectric sensors, wherein the analog front-end processing unit is configured to drive the first light-emitting assembly and the second light-emitting assembly to emit the first optical signal, to receive the photoelectric signals acquired by each of the plurality of photoelectric sensors, and to accordingly obtain testing results corresponding to each blood oxygen measurement channel and testing results corresponding to each heart rate measurement channel; and
a processor, connected to the analog front-end processing unit, wherein the processor is configured to obtain the blood oxygen testing result according to the testing results corresponding to each blood oxygen measurement channel, and to obtain the heart rate testing result according to the testing results corresponding to each heart rate measurement channel.

14. The PPG sensor as claimed in claim 1, wherein
the first light-emitting assembly is a two-in-one LED assembly emitting red light and infrared light, and
the second light-emitting assembly is a green-light LED.

15. The PPG sensor as claimed in claim 14, wherein
the number of the plurality of photoelectric sensors is 4, and
the first light-emitting assembly, the second light-emitting assembly and the four photoelectric sensors constitute four first blood oxygen measurement channels and four heart rate measurement channels.

16. The PPG sensor as claimed in claim 1, wherein
the first light-emitting assembly is a two-in-one LED assembly emitting red light and infrared light, and
the second light-emitting assembly is a three-in-one LED assembly comprising a red-light LED, an infrared-light LED and a green-light LED.

17. The PPG sensor as claimed in claim 16 wherein
the number of the plurality of photoelectric sensors is 4, and
the first light-emitting assembly, the second light-emitting assembly and the four photoelectric sensors constitute eight blood oxygen measurement channels and four heart rate measurement channels.

18. The PPG sensor as claimed in claim 1, wherein
the minimum one of the distances between the second light-emitting assembly and each of the plurality of photoelectric sensors ranges from 4mm to 5mm, and
the distance between the first light-emitting assembly and the at least one of the plurality of photoelectric sensors ranges from 7mm to 9mm.

19. An electronic device, comprising:
a housing, defining a detection window; and
the PPG sensor as claimed in any of claims 1-18, wherein the PPG sensor is exposed from the detection window.

20. A wearable device, comprising:
a strap assembly; and
the electronic device as claimed in claim 19, the strap assembly is configured to fix the electronic device to a human body.
